# EUROPEAN PATENT SPECIFICATION

(11) **EP 4 436 406 B1**
(45) Date of publication and mention of the grant of the patent: **17.06.2026**
(21) Application number: 22822398.8
(22) Date of filing: 25.11.2022
(51) Int. Cl.: A23L 33/105, A23L 33/155, A23L 33/11, A23L 33/15, A61K 45/06, A61P 39/06, A61P 43/00, A61K 31/05, A61K 31/09, A61K 31/122, A61K 31/145, A61K 31/455, A61K 31/592, A61K 31/343

(54) **ANTI-AGEING SUPPLEMENT**
ANTI-AGING-ZUSATZ
COMPLÉMENT ANTI-ÂGE

(30) Priority: 26.11.2021 BE 202105922
(43) Date of publication of application: 02.10.2024
(73) Proprietor: Dormouse BV, 9000 Gent (BE)
(72) Inventor: CRABBE, Ellen, 9870 Zulte (BE)
(74) Representative: Brantsandpatents bv
(86) International application number: PCT/EP2022/083242
(87) International publication number: WO 2023/094580

(56) References cited:
- WO-A1-2016/037971
- WO-A1-2017/109195
- WO-A2-2011/149981
- WO-A2-2012/051591
- US-A1- 2014 023 701
- US-A1- 2015 216 918
- US-A1- 2018 007 945
- LIFE EXTENSION: "NAD+ Cell Regeneration and Resveratrol", 1 January 2018 (2018-01-01), XP002807294, Retrieved from the Internet <URL:https://www.lifeextension.com/vitamins-supplements/item02348/optimized-nad-cell-regenerator-and-resveratrol>

## Description

### TECHNICAL FIELD

The invention relates to nutritional supplements for oral use, as an anti-ageing or longevity agent.

### STATE OF THE ART

Ageing is the changing of organisms as a function of time. As mammals age, certain cellular functions decline. Although the exact mechanisms of ageing have not been fully deciphered, it is clear that there is a link to epigenetic effects on DNA as well as mitochondrial activity in cells. The family of sirtuin proteins is assigned an important role in anti-ageing processes of cells, including through their role in (de)acetylation of DNA and proteins and their link to the intracellular pool of NAD+, an essential co-factor for cell health. When cells age, the cellular pool of NAD+ decreases, also reducing the activity of sirtuins. This causes the ageing of cells and ultimately the organism. The activity of sirtuins is also linked to the activity of mitochondria. The mitochondria are the cell's energy factories, and impairment of the mitochondria, for example by oxidative stress, is also linked to cell ageing.

The current population in Western countries is ageing at a high rate. This means that the number of ageing and affluence diseases such as cardiovascular diseases, as well as diabetes and e.g. Parkinson's disease, is also rising.

The cosmetics industry has a strong focus on ageing. However, this focus is mainly on the visible signs of ageing such as skin ageing. However, these are temporary remedies that essentially do not address or prevent the real ageing of cells.

To date, the pharmaceutical industry has focused on curative treatments for treating ageing symptoms once ageing has set in and not on preventive treatments. This means a huge pressure on the healthcare system, which will keep increasing. This is also recognised by the World Health Organisation and the UN, which have launched initiatives to counter the harmful effects of an ageing population.

There is a need for formulas that have a preventive effect on the ageing of an organism, especially by acting on the cellular mechanisms underlying ageing. In this, it is as yet utopian to create 'eternal life', but it is possible to prolong the active period of humans and animals, and to counteract and delay the symptoms of ageing.

Some components that counteract cellular ageing have already been described. For example, US9795645 describes a composition based on pomegranate extract, which has been described to have an anti-ageing effect. Other disclosures of formulas are discussed in WO2017109195, WO2011149981, US2018007945, US2014023701, US2015216918, WO2012051591 and in Life Extension: "NAD+ Cell regeneration and resveratrol" (www.lifeextension.com).

However, to date, an optimal blend or mix of anti-ageing molecules that counteracts cellular ageing and takes into account the various causes of ageing is lacking.

The invention seeks to solve at least one of the aforementioned problems.

### SUMMARY OF THE INVENTION

The invention relates to a supplement according to claim 1. The composition is optimized such that it positively affects the biological age of a cell and organism.

In particular, the invention relates to an oral anti-ageing composition comprising a mixture of:
- At least one NAD+ precursor chosen from the group of Niacinamide mononucleotide (NMN), Niacinamide Riboside (NR) and niacin
- At least one stilbenoid
- Pomegranate extract and/or ellagitanins
- Sulforaphane or a source of sulforaphane, and
- Vitamin D.

The formulation contains a sophisticated mix of ingredients that respond to the cellular ageing process on multiple fronts, tackling at least one or more of the 9 hallmarks of ageing as described in Lopez-Ortiz et al., 2013.

In a second aspect, the invention relates to a process for slowing down ageing of a subject according to claim 11 and a process for reducing oxidative stress in mitochondria in the cells of a subject according to claim 12. Preferably, the subject concerns a human being.

In particular, the present invention provides a method for slowing down the ageing of a subject, preferably a mammal as well as a method for reducing oxidative stress in mitochondria in the cells of a subject, taking a composition according to the present invention. Due to the specific combination of ingredients, cellular ageing is delayed or prevented at multiple levels, positively affecting the biological age of the subject taking the composition.

### DETAILED DESCRIPTION

Unless otherwise defined, all terms used in the description of the invention, including technical and scientific terms, have the meaning as generally understood by those skilled in the technical field of the invention. For better assessment of the description of the invention, the following terms are explicitly explained.

"A", "the" and "the" refer to both singular and plural in this document unless the context clearly assumes otherwise. For example, "a segment" means one or more than one segment.

The terms "include", "encompass", "comprise", "consist of", "consist of", "provide with", "contain", "contain", "encompassing", "include", "contain", "contain", "contain", "contain" are synonyms and are inclusive or open-ended terms indicating the presence of what follows, and do not exclude or preclude the presence of other components, features, elements, members, steps, known from or described in the prior art.

Citing numeric intervals by the endpoints includes all integers, fractions and/or real numbers between the endpoints, these endpoints included.

Reference to "a carrier" includes a reference to one or more such carriers, and reference to "an excipient" includes a reference to one or more such excipients.

As used herein, 'ageing' and 'senescence' can be used interchangeably to refer to the accumulation of changes that occur over time in a living organism. Such changes can range from those affecting genetic and cellular function to those affecting the function of organs, organ systems or the whole organism. In particular, senescence refers to such changes that occur after an organism has reached biological maturity and may progress to the eventual death of the organism. The term "effects of ageing" herein refers in particular to age-related changes in genetic function, such as changes in transcription of individual genes as well as transcription profiles of groups of genes.

As used herein, the term "biomarkers of ageing" generally refers to genes or gene groups whose expression consistently changes with age, and their transcriptional products, as well as epigenetic effects in the cell. Such genes and gene groups can be called "genetic biomarkers", while the transcriptional products can be called "transcriptional biomarkers". Epigenetic events can be called "epigenetic biomarkers".

As used herein, "up-regulation" and "down-regulation" refer respectively to increased or decreased expression of one or more genes and as a result the protein(s) encoded by those genes, usually in response to some signal or condition. In particular, references to biomarkers of ageing, up-regulation and down-regulation refer to such increases or decreases in response to age, respectively.

As used herein, "formulation" and "composition" may be used interchangeably and refer to a combination of two or more elements or substances. In some embodiments, a composition may include an active agent in combination with a carrier or other excipients, adjuvants, etc.

As used herein, "effective amount" refers to an amount of an ingredient that, when included in a composition, is sufficient to achieve an intended compositional or physiological effect. Thus, a "therapeutically effective amount" refers to a non-toxic but sufficient amount of an active agent to achieve therapeutic results in treating or preventing a condition for which the active agent is known to be effective. Clearly, several biological factors can affect the ability of a substance to perform its intended task. Therefore, an "effective amount" or a "therapeutically effective amount" may in some cases depend on such biological factors. Further, while the achievement of therapeutic effects can be measured by a physician or other qualified medical personnel using assessments known in the technique, it is recognised that individual variation and response to treatments can make the achievement of therapeutic effects a subjective decision.

As used herein, "pharmaceutically acceptable carrier" and "carrier" may be used interchangeably, and refer to any inert and pharmaceutically acceptable material with which a bioactive agent or a nutrient may be combined to obtain a specific dosage formulation for delivery to a subject. As a general principle, carriers must not react with the bioactive agent in a way that substantially degrades or otherwise adversely affects the bioactive agent.

As used herein, "excipient" refers to substantially inert substance, which can be combined with an active agent and a carrier to obtain a specific dosage formulation for delivery to a subject, or to provide a dosage form with specific performance properties. Excipients can include, for example, binders, lubricants, etc. include, but specifically exclude active agents and carriers.

As used herein, "subject" refers to a mammal that may benefit from the administration of a composition or process as disclosed herein. Typically, the subject will be a human. In addition, the subject may also be an animal, preferably a companion animal such as a cat, dog, rabbit, hamster, guinea pig or horse. Where the subject is human, the subject can be of any age. For example, the subject can be 60 years or older, 65 or older, 70 or older, 75 or older, 80 or older, 85 or older, or 90 or older. Alternatively, the subject can be 50 years or younger, 45 or younger, 40 or younger, 35 or younger, 30 or younger, 25 or younger, or 20 or younger.

As used herein, "administration" and "administration" refer to the manner in which an active agent, or composition containing it, is presented to a subject.

Administration can be achieved through various routes known in the technique, such as oral and non-oral methods.

As used herein, "oral administration" refers to a route of administration that can be achieved by swallowing, chewing or sucking an oral dosage form that includes the drug. Examples of known oral dosage forms include tablets, capsules, pills, powders, granules, drinks, syrups, elixirs, confectionery or other foods, etc.

As used herein, the term "aging hallmark" (used synonymously with "age-related hallmark" and "hallmark related to aging") includes, without limitation, any of genomic instability, telomere attrition, an epigenetic alteration, loss of proteostasis, deregulated nutrient sensing, mitochondrial dysfunction, cellular senescence, stem cell exhaustion, and altered intercellular communication. These hallmarks are known, and reviewed in depth by C. Lopez Otin, et al. (7). As used herein, the term "ameliorate", when used in reference to an aging hallmark, includes, without limitation, (i) slowing, stopping, reversing, or preventing the hallmark's progression, (ii) slowing, stopping, reversing, or preventing the progression of the hallmark's symptoms, (iii) preventing or reducing the likelihood of the hallmark's recurrence, and/or (iv) preventing or reducing the likelihood that the hallmark's symptoms will recur. In one embodiment, treating a subject afflicted with an aging hallmark means (i) reversing the hallmark's progression, ideally to the point of eliminating the hallmark, and/or (ii) reversing the progression of the hallmark's symptoms, ideally to the point of eliminating the symptoms.

In a first aspect, the invention relates to an oral anti-ageing composition. Anti-ageing refers to the process by which the ageing process of an organism is slowed or prevented. Ageing can be reflected in external characteristics such as getting grey hair or hair loss, the reduction of skin elasticity and/or collagen, getting wrinkles but also in internal characteristics such as an effect on cellular health such as on mitochondria, epigenetic effects, the telomeres, molecular markers linked to ageing... as well as, for example, a reduction in bone density, muscle mass....

In a preferred form, the composition includes a mixture of:
- At least one NAD+ precursor chosen from the group of Niacinamide mononucleotide (NMN), niacinamide riboside (NR) and niacin
- Resveratrol and/or pterostilbene
- Pomegranate extract and/or ellagitanins
- Sulforaphane or a source of sulforaphane, and
- Vitamin D.

The composition consists of ingredients, each of which has already been attributed a positive role on the anti-ageing process. The composition has been optimised such that it has a synergistic effect and will optimally inhibit the ageing process of a subject, preferably a human or animal. More specifically, the composition has been shown to have optimal action on mitochondria and their density in the cell. In addition, when taken daily orally or on a very regular basis, the composition also appears to have a positive effect on molecular and/or epigenetic markers known to be involved in the ageing process. A test population taking the supplement reported a marked improvement in vitality and general well-being.

In a further preferred form, the composition also includes vitamin K and/or trimethylglycine, also known as betaine.

In particular, the present invention relates to an oral anti-ageing composition, wherein an effective dose of the composition includes
- At least 500 mg per 100 g composition Niacinamide mononucleotide (NMN), niacinamide riboside (NR) and/or niacin
- At least 1250 mg per 100 g of composition resveratrol and/or a pterostilbene
- At least 5000 mg per 100 g composition pomegranate extract and/or ellagitanins
- At least 100 mg per 100 g composition sulforaphane or a source of sulforaphane
- At least 750 µg per 100 g of composition Vitamin D.

NMN, NR and niacin are known precursors of NAD+. As an organism ages, the levels of NAD+ go down. NAD+ is involved not only in metabolism, but also in DNA repair, regulation of gene expression and signal transduction. NAD+ is a co-factor of sirtuins, proteins whose role in ageing has been described in detail. As the amount of cellular NAD+ decreases during the ageing process, the function of the sirtuins is also reduced. This has a major impact on the ageing process of cells and the body. Providing a precursor of NAD+ ensures that the cellular level of NAD+ remains sufficiently high. Both NR, NMN and niacin (vitamin B3) are suitable for that purpose.

In a preferred form, the NAD+ source is niacin or vitamin B3. Niacin is safe for human use and supplementation of niacin was found to have a positive impact on mitochondrial myopathy, among others.

The total amount of NR, NMN and/or niacin in the composition is preferably at least 500 mg per 100 g composition, more preferably at least 600 mg, 700 mg, 800 mg, 900 mg, 1,000 mg per 100 g composition. The total amount of NR, NMN and/or niacin in the composition is preferably between 500 mg and 40,000 mg per 100 g composition, more preferably between 500 mg and 30,000 mg, more preferably between 750 mg and 30,000 mg per 100 g composition.

The NR, NMN or niacin content can be increased by the additional addition of natural sources of the latter such as buckwheat sprouts, green leaves, chia, sunflower seeds or brown rice. Preferably, the composition is enriched with ground buckwheat germs (also known as buckwheat germ meal).

The composition or formulation according to the present invention also comprises a stilbenoid, preferably chosen from the group of (trans-)resveratrol and/or pterostilbene or a derivative thereof. These polyphenols, present in red grapes, blueberries and dark chocolate, among others, are activators of sirtuins. Mice fed with resveratrol showed a longer lifespan than control mice and were also generally in better shape and more active. Stilbenoids such as (trans-)resveratrol and pterostilbene also play a role in preventing diseases of affluence such as diabetes and obesity.

In a preferred form, the composition comprises at least 1250 mg per 100 g of composition (trans) resveratrol and/or pterostilbene. More preferably, the composition comprises at least 1500 mg per 100 g, more preferably at least 1750 mg per 100 g, more preferably at least 2000 mg per 100 g. In another or further preferred form, the composition comprises between 1250 mg and 3000 mg per 100 g composition (trans)resveratrol and/or pterostilbene, more preferably between 2000 and 2750 mg per 100 g composition.

In an embodiment, the composition comprises at least 5000 mg per 100 g composition pomegranate extract and/or ellagitannins and/or urolithin A. Pomegranate extract includes ellagitannins, precursors of urolithin A, a component whose positive influence on mitochondria has been described. When an organism ages, the amount of mitochondrial activity goes down. Supplementation with pomegranate extract has a positive impact on mitochondrial health. Pomegranate also a high source of punicalagin, a specific ellagitannin that plays a role in preventing collagen degradation and skin ageing. Extracts of pomegranate have been described in prior art. Preferably, an extract containing at least 40% punicosides is used. In a further preferred embodiment, the composition comprises at least 6000 mg, at least 7500 mg, at least 8500 mg, at least 9000 mg, at least 10,000 mg per 100 g composition pomegranate extract and/or ellagitannins and/or urolithin A. In another or further embodiment, the composition comprises between 5000 and 20,000 mg per 100 g composition pomegranate extract and/or ellagitannins, more preferably between 7500 and 15,000 mg per 100 g composition pomegranate extract and/or ellagitannins.

The composition comprises sulforaphane or a source of sulforaphane. In an embodiment, the source of sulforaphane is broccoli, more preferably broccoli sprouts or an extract thereof. In an embodiment, the composition comprises at least 1000 ppm sulforaphane, more preferably at least 2000, more preferably at least 2500 ppm sulforaphane. In another or further embodiment, the composition comprises between 1000 and 5000 ppm sulforaphane.

In the embodiment in which the sulforaphane content is provided via a source of sulforaphane, such as broccoli sprouts, the composition preferably comprises at least 5,000 mg per 100 g composition broccoli sprouts or a concentrate of broccoli sprouts, more preferably at least 6,000 mg, more preferably at least 7,000 mg per 100 g composition broccoli sprouts or a concentrate of broccoli sprouts. In another or further embodiment, the composition comprises between 5000 and 10,000 mg per 100 g composition broccoli sprouts or a concentrate of broccoli sprouts.

The formulation further includes vitamin D. The importance of supplementation with vitamin D became once again evident with the covid-19 pandemic that broke out in 2020. Low levels of vitamin D are associated with numerous clinical complications such as osteoporosis and a general decline in physical and mental abilities. These symptoms increase as an individual's age increases. Vitamin D can be added to the composition as vitamin D3 or vitamin D2 or a mixture of them. Vitamin D plays a vital role in human health. Vitamin D deficiency can have a drastic impact on both the physical and mental health of a subject. Vitamin D deficiency has been linked to health problems such as cognitive decline, depression, cardiovascular disease, hypertension, diabetes and cancer. As individuals age, the risk of vitamin D deficiency increases significantly. The percentage of older adults suffering from vitamin D deficiency ranges from 20 to 100 per cent in the United States (US). Risk factors contributing to vitamin D deficiency in older adults include reduced dietary intake of vitamin D, increasing adiposity, reduced cutaneous synthesis of vitamin D and spending less time outdoors. When a subject ages, the immune system also goes downhill. This natural process is known as immunosenescence. The immune system of an older adult gradually loses its ability to fight infections and develop active immunity after vaccinations, leading to an increase in mortality rates in the elderly [16]. Vitamin D helps regulate innate and adaptive immune responses through T-cell differentiation and expression of cathelicidin, a protein that kills infectious organisms.

In one embodiment, the composition comprises at least 750 µg per 100 g composition Vitamin D, more preferably at least 1000 µp per 100 g composition Vitamin D. In another or further preferred embodiment, the composition comprises between 750 and 1500 µg Vitamin D per 100 g composition.

The vitamin D in the composition is preferably vitamin D2 or vitamin D3; more preferably Vitamin D3.

In addition to the key components, the composition may also include some further supporting components that play a role in the cellular ageing process.

In one embodiment, the composition includes betaine or trimethylglycine (TMG). TMG aids in the methylation of proteins, including histones. TMG was found to have a positive effect on liver detoxification. In addition, TMG also helps in the conversion of homocysteine to methionine. High levels of homocysteine have been associated with an increased risk of heart disease, heart attacks, strokes, Alzheimer's disease, Parkinson's disease and osteoporosis. Through its activity as a methyl donor, TMG can further ensure that the methyl pool in our body remains optimal, especially when supplemented with niacin, which is known to be a methyl consumer.

In one embodiment, the composition comprises between 5,000 and 10,000 mg per 100 g of composition TMG.

In one performance form, its composition includes vitamin K. Vitamin K is an often ignored vitamin. Studies show that vitamin K supplementation can have a beneficial effect on diseases related to ageing, such as osteoarthritis, rheumatoid arthritis and cardiovascular disease. Moreover, vitamin K can affect mitochondrial health.

In one embodiment, the composition includes between 100 and 500 µg of vitamin K per 100 g of composition.

In an embodiment, a composition according to the current invention may comprise alpha-ketoglutarate. Alpha-ketoglutarate, also known as alpha-ketoglutaric acid, is commercially available. Alpha-ketoglutarate is available as the calcium salt, but all other salts of alpha-ketoglutarate are envisioned in this invention. In embodiment of the current invention, alpha-ketoglutarate is present in an amount of between 500 mg to 20.000 mg alpha-ketoglutarate per 100 g composition, more preferably between 1.000 and 15.000 mg alpha-ketoglutarate per 100 g composition.

Compositions according to the present invention may additionally and/or optionally further include one or more free amino acids or peptides, such as lysine, methionine, histidine, leucine, isoleucine, alanine, phenylalanine asparagine arginine, beta-alanine, aspartic acid, tryptophan, proline, threonine, cysteine, selenocysteine, serine, taurine, tyrosine, valine, glycine, glutamine, glutamic acid, ornithine, carnosine, L-carnitine, glutathione.

Compositions according to the present description additionally and/or optionally further contain one or more minerals selected from the group of calcium, sodium, potassium, phosphorus, chromium, vanadium, magnesium, zinc, manganese, selenium, copper, molybdenum, boron, vanadium, iron (and/or derivatives) or a combination thereof.

According to an embodiment of the invention, the composition comprises an orally administerable dosage form. Examples of an orally administerable dosage form include, but are not limited to, a tablet, capsule, powder that can be dispersed in a beverage, a liquid such as a solution, suspension or emulsion, a soft gel/chew capsule, a lozenge tablet, a paste, elixir, syrup, drops, bar), beverage, chewable tablet, a chewable bar or any other suitable dosage form known in the technique. Preferably, the composition is formulated in powder form, as a tablet, capsule or granule. Additionally, the orally administered dosage forms may be formulated for immediate release, extended release or delayed release.

In a preferred form, the composition is a powder and a daily dose is preferably located between 2 and 20 grams, more preferably between 3 and 10 grams, more preferably between 3 and 7 grams. The composition may be packaged in bulk, or may be packaged per daily dose, in a sachet or stick.

Compositions according to the present description may additionally and/or optionally further include one or more excipients. These excipients, are selected from the following group: microcrystalline cellulose, crystalline cellulose, lactose, maize starch, sucrose, glucose; binders such as tragacant, gum arabic, maize starch, gelatine, polyvinyl alcohol, polyvinyl ether, ethyl cellulose, methyl cellulose, shellac, hydroxypropyl cellulose, hydroxypropyl starch, polyvinylpyrrolidone; swelling agents such as maize starch, pregelatinised starch, alginic acid, dextrin; lubricants such as magnesium stearate; fluidity enhancers such as fine silica; lubricants such as glyceryl fatty acid ester, magnesium stearate, talc, polyethylene glycol, silica, hydrogenated vegetable oil sweeteners such as sucrose, lactose, aspartame, acesulfame-K, sucralose, monatin, stevia, saccharin and the like; flavourings to be used for various foods such as peppermint, vanilla flavour, cherry, raspberry ketone and the like; preservatives such as paraoxybenzoates, chlorobutanol, benzyl alcohol, sorbic acid; an antioxidant such as sulphite, ascorbic acid, vitamin E, butylated hydroxytoluene, sodium sulphite; and/or coating agents such as shellac, sucrose, gelatine and hydroxypropyl cellulose.

Composition according to the present description may additionally and/or optionally include further phytochemical molecules derived from at least one of the following sources: bilberry, cranberry, raspberry, cherry, mulberry, maqui berry, purple corn, strawberry, grapes, gooseberries, blackcurrants, grape must, cocoa beans, coffee beans, pine bark, cardamom, cinnamon bark, ginseng, astragalus, rhodiola, garcinia, ginger, ginkgo, citrus fruits, grape skins, grape seeds, hawthorn, artichoke, apple, olive, orange, lemon, pepper, soy, mango, tea leaves, tomato, cabbage, black rice, bitter lemon, stevia, lo han, goji (wolf berry), sea buckthorn, kudzu, cloves, hemp, cassia, magnolia, nutmeg, jujube, honeysuckle, poria, bellflower, lotus, basil, sesame, angelica, cimicifuga, epimedium, schisandra, salvia, liquorice, privet, ophiopogonis, aloe, dodder, fenugreek, gotu kola, guarana, purslane and tribulus.

Compositions according to the present description may additionally and/or optionally further include one or more additional natural extracts chosen from one or more plants of the following genera: Origanum Thymus, Lavandula, Salvia, Melissa, Cuminum, Petroselinum, Calendula, Tagetes, Boswellia, Sambucus, Copaifera, Allium, Symphytum, Punica, Euterpe, Sophora, Rheum, Fagopyrum, Camellia, Coptis, Hydrastis, Mahonia, Phellodendron, Berberis, Xanthorhiza, Lonicera, Vaccinium, Cinnamomum, Vitis, Terminalia, Pinus, Albizia, Melia, Salvadora, Paullinia, Commiphora, Juglans, Scutellaria and Magnolia

In particular, the additional natural extract used in the compositions described herein may be extracted from plants of the following species: Origanum vulgare, Origanum onites, Origanum majorana, Origanum heracleoticum, Thymus vulgaris L, Thymus citriodorus, Thymus pulegioides, Thymus x herba-barona , Thymus serpyllum, Lavandula angustifolia / officinalis, Lavandula stoechas, Lavandula dentate, Lavandula x intermedia, Lavandula multifida, Salvia officinalis, Salvia divinorum, Salvia apiana, Melissa officinalis, Cuminum cyminum, Petroselinum crispum, Calendula arvensis, Calendula maderensis, Calendula officinalis, Tagetes erecta, Tagetes minuta, Tagetes patula, Boswellia sacra, Boswellia frereana, Boswellia serrata, Boswellia papyrifera, Sambucus nigra, Sambucus melanocarpa, Sambucus racemosa, Copaifera langsdorfii, Curcuma longa, Allium sativu, Symphytum officinale, Punica granatum, Euterpe oleracea, Sophora flavescens, Rheum rhabarbarum, Rheum rhaponticum, Fagopyrum esculentum, Camel lia sinensis, Coptis teeta, Hydrastis canadensis, Mahonia aquifolium, Phellodendron amurense, Berberis vulgaris, Xanthorhiza simplicissima, Lonicera ceprifoliu, Vaccinium macrocarpon, Cinnamomum zeylanicum Nees, Cinnamomum verum, Vitis Vinifera, Terminalia Bellerica, Pinus Pinaster, Albizia Lebbek, Melia Azadirachta, Salvadora persica, Paullinia cupana, Piper betle, Commiphora myrrha, Juglans regia, Scutellaria baicalensis and Lombardia officinalis.

Additional natural extracts that may be useful together with previously described active ingredients may also be chosen from one or more of the following natural extracts (general name included first, not italicised, followed by official italicised name(s)): achyranthes, Achyranthes aspera, aloe, Aloe spp., includingA. barbadensis, A. feroxandA. vera, aniseed, Pimpinella anisum, aristolochia, Aristolochia bracteolate, arnica, Arnica spp, includingA. fulgens, banyan, Ficus bengalensis, bakula, Mimusops elengi, basil, Ocimum basilicumandO. minimum, betel, Piper betle, camphor, camphor, Cinnamomum camphora, catechu, Acacia catechu, stinking goldenrod, Chelidonium spp, chamomile, Matricaria chamomilla, chebula, Terminalia chebula, Chinese calotte, Scutellaria baicalensis, cinnamon, Cinnamomum lourerii and C. zeylandicum, citrus, Citrus spp. including C.aurantifolia, C. aurantium, C. limonumandC. sinensis, dill, Anethum spp, includingA. graveolensandA. sowa, echinacea (sunflower), Echinacea pallida, eucalyptus, Eucalyptus globulus, fennel, Foeniculum vulgare, gardenia, Gardenia jasminoides, grape, Vitis vinifera, hops, Humulus lupulus, wood tuynia, Houttuynia cordata, Indian mulberry, Morinda citrifolia, juniper, Juniperus communis , lemongrass, Cymbopogon spp, includingC.citratusandC.flexuosus, liquorice, Glycyrrhiza spp, including G.labraandG.uralensis, long pepper (pipli), Piper longum, madhuca, Madhuca longifolia, magnolia, Magnolia officinalis, marigold, Calendula officinalis, mastic, Pistacia lentiscus, melilot, Melilotus officinalis, yarrow, Achillea millefolium, myrrh, Commiphora spp. neem (margosa), Azadirachta indica, neroli (bitter orange blossom), Citrus aurantium, nutmeg, Myristica fragrans, oak gall, Quercus infectoria, parsley, Petroselinum sativum, peelu, Salvadora persica, peppermint Mentha piperita, pine, Pinus spp., includingP. palustrisandP. sylvestris, pomegranate, Punica granatum, prickly acacia (babul), Acacia nilotica, rhatany, Krameria spp., includingK argentaandK triandra, rosemary, Rosmarinus officinalis, saffron, Crocus sativus, sage, Salvia spp., includingS. lavendulaefolia, S. officinalisandS.triloba, sandalwood, Santalum spp, includingS.albumandS.spicatum, spearmintMentha spicata, spilanthes (akarkara), Spilanthes calvi, star anise, Illicium verum, tea (including green tea and oolong tea), Camellia sinensis, thyme, Thymus spp, includingT. serpyllumandT vulgaris, tomar (prickly ash), Zanthoxylum armatum, tulsi (holy basil), Ocimum sanctum, usnea, Usnea barbata, vajradanti, Potentillafulgens, walnut, Juglans regia, wintergreen, Gaultheria procumbens and mixtures thereof.

As discussed herein, the additional natural extracts may be derived from or based on compounds or extracts isolated from plants.

Composition and formulations according to the present description may additionally and/or optionally further include one or more vitamins selected from the group: vitamin A, vitamin C, vitamin B1, vitamin B2, vitamin B5, vitamin B6, vitamin B12, vitamin D, vitamin E, and/or derivatives), folic acid, biotin.

Compositions according to the present invention may additionally and/or optionally further comprise one or more additives, such as fruit juice, dextrin, cyclic oligosaccharide, cyclodextrins (alpha, beta, gamma), saccharides (monosaccharides such as fructose, glucose and/or polysaccharides), buffers, acidulant, flavouring, Hiki cha powder or other flavour modifiers, an emulsifier, collagen, milk powder, polysaccharide thickener, agar or other texture modifiers, at least one vitamin, eggshell calcium, calcium pantothenate, other minerals, royal jelly, propolis honey, dietary fibre, Agaricus subrufescens, chitin, chitosan, flavonoids, carotenoids, lutein, herbal medicine, chondroitin and/or amino acids.

Compositions according to the present description may also include creatine and its derivatives, capsate, carnitine, nordihydrocapsate, polyphenols and agmatine (or agmatine sulphate).

Compositions according to the present invention may additionally and/or optionally further comprise one or more citrus bioflavonoids, (such as quercetin, rutin, isoquercetin, synephrine and octopamine); CoQ10, thiamine, citrulline malate, citrulline, lutein, lycopene, capsaicin, arginine alpha ketoglutarate (Arginine AKG), L-arginine pyroglutamate, arginine ketoisocaproate, citric acid, ornithine alpha ketoglutarate (Ornithine AKG), omega-3 fatty acids (DHA and EPA), L-norvaline, nitrate, taurine, arginine ethyl ester, carnosine, vanadyl sulphate, L-alpha glycerophosphorylcholine (Alpha GPC), Pinus pinaster (Pycnogenol^{®}), rutacaerpine, Epimedium spp., garlic (allicin, alliin and the like), flaxseed, flaxseed legumes (alpha-linolenic acid (ALA), gamma-linolenic acid (GLA)), Schisandria (as as Schisandrin, Schisandrol A and B and Gamma schisandrin), green tea catechins (as catechin, ECGC, ECG and EGC), black tea dong quai (ligustilide), Andrographis (Andrographolides), grape extract (such as resveratrol) anthocyanins (such as cyanidin 3-glucosi de (C3G), cyanidin 3-rutinoside, delphinidin 3-glucoside and malvidin 3-glucoside), Danshen, Beta vulgaris root, celery (3-N-butylphthalide), Berberine, Motherwort, Jasmine, Lemon balm, vinpocetine, Lotus, Malrove, Lemongrass, Yerba mate, Peony, Mustard, Motherwort, Cramp bark, Grapeseed, Proanthocyanidins (PACs, including Procyanidin A1, Procyanidin A2, Procyanidin B1, Procyanidin B2, and the like), Spinach (with nitrates), Kale (with nitrates), Beet (with nitrates), Theobromine, theophylline, phenylethylamine (PEA) and the like), Hawthorn, Hawthorn flavonoids (hyperoside, vitexin, isovitexin and the like), Catauba extract, apple polyphenols and combinations thereof.

Compositions and formulations according to the present invention may additionally and/or optionally further comprise one or more carrier materials, such as corn starch, acacia, gelatin, malt, tragacant, microcrystalline cellulose, kaolin, dicalcium phosphate, calcium carbonate, sodium chloride or alginic acid disintegrating agents including microcrystalline cellulose or alginic acid; binders including acacia, methylcellulose, ethylcellulose, sodium carboxymethylcellulose, polyvinylpyrrolidone or hydroxypropylmethylcellulose; and lubricants such as magnesium stearates, stearic acid, silicone liquid, talc, oils, waxes or colloidal silica.

Compositions and formulations according to the present invention may additionally and/or optionally further comprise one or more mixtures of acids, (such as citric, tartaric, malic and fumaric acids or a combination thereof) and carbonates such as sodium, potassium bicarbonate or carbonate; water-soluble binders (starches, natural gums, cellulose gum, microcrystalline cellulose, methyl cellulose, cellulose ethers, ethyl cellulose, sodium carboxymethylcellulose, gelatin, dextrose, lactose, sucrose, sorbitol, mannitol polyethylene glycol, polyvinylpyrrolidone, pectins, alginates, polyacrylamides, polyvinyloxoazolidone, polyvinyl alcohols and mixtures thereof) and/or lubricants (sodium benzoate, polyethylene glycol, L-leucine, adipic acid and combinations thereof).

Compositions and formulations according to the present invention may additionally and/or optionally further comprise one or more other ingredients, including, for example, flavourings, fillers, surfactants, colourings and sweeteners.

Compositions and formulations according to the present invention may additionally and/or optionally further comprise one or more other water or other aqueous formulations, including suspending agents such as, for example, alginates, pectin, kelgin, carrageenan, acacia, methylcellulose, polyvinyl alcohol or polyvinylpyrrolidone.

Compositions and formulations according to the present invention may additionally and/or optionally further include one or more, aqueous or non-aqueous carriers, diluents, fillers, binders, wetting agents, disintegrants, solvent retardants, absorption accelerators wetting agents, absorbers or lubricants, solvents or carriers, including water ethanol, polyols (propene glycol, polyethylene glycol or glycerol), suitable mixtures thereof, vegetable oils (such as olive oil) and organic esters such as ethyl oleate.

Furthermore, compositions and formulations according to the present description may include stabilisers, solubilising agents, suspending agents, emulsifiers, sedatives, buffers, preservatives, isotonic agents or antibacterial and antifungal agents.

Finally, compositions and formulations according to the present invention may also include pre- and/or probiotics, such as there are oligosaccharides, fibres, Bifidobacterium longum, Bifidobacterium longum lactis, Bifidobacterium bifidum, Bifidobacterium breve, Bifidobacterium longum infantis, Bifidobacterium longum, Lactobacillus helveticus, Lactobacillus acidophilus, Lactobacillus casei, Lactobacillus delbrueckii subsp or any other Lactobacillus sp. whose beneficial effects have been described.

In a second and further aspect, the present invention relates, on the one hand, to a process for slowing down ageing of a subject as well as a method for reducing oxidative stress in mitochondria in the cells of a subject, wherein a composition according to one of the embodiments described above is taken orally each time. In a preferred embodiment, the composition is taken once or twice daily with a liquid, more preferably the composition is dissolved or dispersed in a liquid for absorption. In a further preferred form, the composition is administered in tablet form and taken with a liquid.

The composition has a positive effect on mitochondria in cells and thus on overall cellular health, and also affects the biological age of the subject taking the composition for a prolonged period (at least three months). Preferably, the composition is taken with a liquid, more preferably dissolved in a liquid before ingestion. Specifically, the subject will be a human, or a mammal, preferably chosen from the group of companion animals such as a dog, cat, mouse, halmster, guinea pig or horse.

In an embodiment, a daily dose or 1 serving of the composition includes:
- at least 25 µg of a NAD+ precursor chosen from the group of Niacinamide mononucleotide (NMN), Niacinamide Riboside (NR) and niacin
- at least 100 mg of a stilbenoid, preferably chosen from the group of (trans) resveratrol or pterostilbene
- at least 500 mg pomegranate extract
- at least 250 mg of broccoli sprouts, and
- at least 50 µg of Vitamin D.

In an embodiment a daily dose or 1 serving of the composition includes:
- between 25 µg and 70 µg of a NAD+ precursor chosen from the group of Niacinamide mononucleotide (NMN), Niacinamide Riboside (NR) and niacin
- between 100 mg and 200 mg of a stilbenoid, preferably chosen from the group of (trans) resveratrol or pterostilbene
- between 500 mg and 1000 mg pomegranate extract
- between 250 mg and 1000 mg of broccoli sprouts, and
- between 50 µg and 100 µg of Vitamin D.

In a further embodiment, the daily dose or 1 serving comprises at least 250 mg TMG, more preferably between 250 and 750 mg TMG.

In a further or other embodiment, the daily dose or 1 serving comprises at least 10 µg of vitamin K, more preferably between 10 and 20 µg of vitamin K.

These amounts are determined such that they are effective and cause no significant side effects.

In an embodiment, at least one of the parameters chosen from the group consisting of genomic instability, telomere attrition, an epigenetic alteration, loss of proteostasis, deregulated nutrient sensing, mitochondrial dysfunction, cellular senescence, stem cell exhaustion, and altered intercellular communication is measured, as a marker of the ageing process.

The success of the present composition in ameliorating epigenetic dysregulation in a subject (e.g., a human subject) can be measured, for example, by making any of the following determinations: (i) the subject's epigenetic methylation, histonylation, and/or chromatin patterns more closely resemble the patterns of younger persons (e.g., by at least 1%, at least 2%, at least 5%, at least 10%, at least 20%, at least 30%, at least 40%, at least 50%, at least 60%, at least 70%, at least 80%, at least 90%, or at least 100%); (ii) an epigenetic clock (e.g., a Horvath clock or a Levine clock)-measuring methylation of DNA in different regions of the subject's DNA more closely resembles the DNA methylation pattern of a younger subject (e.g., by at least 1 %, at least 2%, at least 5%, at least 10%, at least 20%, at least 30%, at least 40%, at least 50%, at least 60%, at least 70%, at least 80%, at least 90%, or at least 100%); (iii) an epigenetic DNA methylation clock (e.g., a Horvath clock or a Levine clock) shows that an adult human subject has been rejuvenated by at least one year, at least two years, or at least three years after being administered the present composition (or of which the epigenetic age or biological age has been decreased by at least 1%, at least 2%, at least 3%, at least 5%, at least 10%, at least 20%, at least 30%, at least 40%, at least 50%, at least 60%, at least 70%, at least 80%, at least 90%, or at least 100% compared to a subject's chronological age); (iv) the subject's chromatin distribution more closely resembles that of a younger subject (e.g., by at least 0.2%, at least 1 %, at least 2%, at least 5%, at least 10%, at least 20%, at least 30%, at least 40%, at least 50%, at least 60%, at least 70%, at least 80%, at least 90%, or at least 100%); (v) the subject's transcriptome resembles the transcriptome of a younger subject (e.g., by at least 0.2%, at least 0.5%, at least 1%, at least 2%, at least 5%, at least 10%, at least 20%, at least 30%, at least 40%, at least 50%, at least 60%, at least 70%, at least 80%, at least 90%, or at least 100%); (vi) the subject's expression profile of ribosome-related proteins and/or ribosome-related RNA more closely resembles that of a younger organism (e.g., by at least 0.2%, at least 0.5%, at least 1%, at least 2%, at least 5%, at least 10%, at least 20%, at least 30%, at least 40%, at least 50%, at least 60%, at least 70%, at least 80%, at least 90%, or at least 100%); or (vii) the subject's levels of sirtuins have increased (e.g., by at least 1%, at least 2%, at least 5%, at least 10%, at least 20%, at least 30%, at least 40%, at least 50%, at least 60%, at least 70%, at least 80%, at least 90%, or at least 100%).

The success of the present composition in ameliorating epigenetic dysregulation in a subject (e.g., a human subject) can also be measured, for example, by making any of the following determinations: (i) an increase in the level of H3K9me3 in the subject (e.g., by at least 1%, at least 2%, at least 5%, at least 10%, at least 20%, at least 30%, at least 40%, at least 50%, at least 60%, at least 70%, at least 80%, at least 90%, or at least 100%); or (ii) an increase in the level of heterochromatin protein (Hp1y) in the subject (e.g., by at least 1%, at least 2%, at least 5%, at least 10%, at least 20%, at least 30%, at least 40%, at least 50%, at least 60%, at least 70%, at least 80%, at least 90%, or at least 100%).

The success of the present composition in ameliorating cell senescence in a subject (e.g., a human subject) can be measured, for example, by making any of the following determinations: (i) a delay in the occurrence of senescent cells or a delay in the transition of normal cells into senescent cells in vitro or in vivo (e.g., by at least 2%, at least 5%, at least 10%, at least 20%, at least 30%, at least 40%, at least 50%, at least 60%, at least 70%, at least 80%, at least 90%, or at least 100%); and/or (ii) a decrease in the number of senescent cells in vitro or in vivo (e.g., by at least 1 %, at least 2%, at least 5%, at least 10%, at least 20%, at least 30%, at least 40%, at least 50%, at least 60%, at least 70%, at least 80%, at least 90%, or at least 100%). Senescence or senescent-like status of cells can be measured, for example, via one or more of the following: (i) epigenetic profiling such as measuring epigenetic markers of senescence (e.g., changes in histone H3 lysine 9 and 27 trimethylation (changed levels of H3K9me3 and H3K27me3), changed heterochromatin protein 1 (HP1) family protein levels, increased histone variant macroH2A levels, and/or chromatin remodeling enzyme ATRX levels); (ii) changes in cell morphology resembling the morphology of senescent cells, (e.g., enlarged size, a more flattened shape, polyploid nuclei, or accumulation of DNA damage foci); (iii) changes in levels of nuclear lamina-associated proteins such as lamins (e.g., a decline lamin B1); (iv) heterochromatin changes more resembling the heterochromatin status of senescent cells (e.g., an increase in senescence-associated heterochromatic foci (SAHF)); (v) an increase of senescence-associated distention of satellites (SADS) or other changes in pericentric satellite DNA more resembling that of senescent cells; and (vi) changes in secretory phenotype corresponding to that of senescent cells (e.g., the occurrence of a more senescence-associated secretome including, without limitation, cytokines, interleukins (e.g., IL-1, IL-2, IL-6, II-8, and TNF- alpha), matrix metalloproteinases, pro angiogenetic factors, pro-inflammatory substances, and growth factors (e.g., vascular endothelial growth factor, insulin like growth factor, and GM-CSF)).

The success of the present composition in ameliorating cell senescence in a subject (e.g., a human subject) can also be measured, for example, by making any of the following determinations: (i) an increase in the level of H3K9me3 in the subject (e.g., by at least 5%, at least 10%, at least 20%, at least 30%, at least 40%, at least 50%, at least 60%, at least 70%, at least 80%, at least 90%, or at least 100%); (ii) an increase in the level of H3K27me3 in the subject (e.g., by at least 5%, at least 10%, at least 20%, at least 30%, at least 40%, at least 50%, at least 60%, at least 70%, at least 80%, at least 90%, or at least 100%); (iii) a decrease in the level of P16INK4A in the subject (e.g., by at least 5%, at least 10%, at least 20%, at least 30%, at least 40%, at least 50%, at least 60%, at least 70%, at least 80%, at least 90%, or at least 100%); or (iv) a decrease in the level of one or more of p16, p53, p21, p14, p15, and beta-galactosidase activity in the subject (e.g., by at least 5%, at least 10%, at least 20%, at least 30%, at least 40%, at least 50%, at least 60%, at least 70%, at least 80%, at least 90%, or at least 100%).

The success of the present composition in ameliorating altered intercellular communication in a subject (e.g., a human subject) can be measured, for example, by determining a decrease in the level of one or more of IL-1, IL-2, IL-6, IL-8, IL-17a, and TNF-alpha in the subject (e.g., by at least 5%, at least 10%, at least 20%, at least 30%, at least 40%, at least 50%, at least 60%, at least 70%, at least 80%, at least 90%, or at least 100%).

The success of the present composition in ameliorating decreased autophagy in a subject (e.g., a human subject) can be measured, for example, by determining any of the following: (i) an increase in the level of one or more of LC3, p62, Ulk1 , Parkin, PINK1 , LAMP2A, Atg, FIP200, Vps15, Beclin, Lamp-1 , Lamp-2, Hsp70, Hsp90, and SQSTM1 in the subject (e.g., by at least 5%, at least 10%, at least 20%, at least 30%, at least 40%, at least 50%, at least 60%, at least 70%, at least 80%, at least 90%, or at least 100%); (ii) an increase (e.g., by at least 5%, at least 10%, at least 20%, at least 30%, at least 40%, at least 50%, at least 60%, at least 70%, at least 80%, at least 90%, or at least 100%) in any of phagophore formation, autophagosome formation, lysosome numbers, lysosomal activity, LC3 puncta, lysosomal content (as determined, for example, using immunochemistry, electron microscopy, Western blotting, or flow cytometry); (iii) increased lysosome acidity (e.g., by at least 5%, at least 10%, at least 20%, at least 30%, at least 40%, at least 50%, at least 60%, at least 70%, at least 80%, at least 90%, or at least 100%); (iv) upregulation of the ubiquitin-proteasome system (e.g., by at least 5%, at least 10%, at least 20%, at least 30%, at least 40%, at least 50%, at least 60%, at least 70%, at least 80%, at least 90%, or at least 100%); or (v) inhibition of mTOR or the mTOR pathway (e.g., by at least 5%, at least 10%, at least 20%, at least 30%, at least 40%, at least 50%, at least 60%, at least 70%, at least 80%, at least 90%, or at least 100%).

The success of the present composition in ameliorating telomere attrition in a subject (e.g., a human subject) can be measured, for example, by making any of the following determinations: (i) an increase in telomere length in the subject (e.g., by at least 0.01%, at least 0.1%, at least 0.5%, at least 1.0%, at least 5%, at least 10%, at least 20%, at least 30%, at least 40%, or at least 50%); or (ii) an increase in mRNA expression of telomerase, TERT, TERC, or telomerase-related genes (e.g., by at least 1%, at least 2%, at least 5%, at least 10%, at least 20%, at least 30%, at least 40%, at least 50%, at least 60%, at least 70%, at least 80%, at least 90%, or at least 100%).

The success of the present composition in ameliorating stem cell exhaustion in a subject (e.g., a human subject) can be measured, for example, by determining an increase in the number of stem cells in vivo (e.g., by at least 1%, at least 2%, at least 5%). This increase can be measured in vitro or in vivo via, for example, one or more of the following biomarkers (i) muscle satellite stem cell biomarkers (e.g., PAX7, LMNA, and emerin); (ii) neuronal stem cell biomarkers (e.g., Nestin, Sox2, ASCL1/Mash1, ABCG2, BMI-1, beta-Catenin, Brg1, N-Cadherin, Calcitonin R, CD15/Lewis X, CD133, CDCP1 , COUP-TF I/NR2F1 , CXCR4, FABP7/B-FABP, FABP8/M-FABP, FGFR2, FGFR4, FoxD3, Frizzled-9, GCNF/NR6A1 , GFAP, Glutl, HOXB1 , ID2, LRTM1 , Meteorin, MSX1, Musashi-1, Musashi-2, Nestin, NeuroDI, Noggin, Notch-1, Notch-2, Nrf2, Nucleostemin, Numb, Otx2, Pax3, Pax6, PDGF R alpha, PKC zeta, Prominin 2, ROR2, RUNX1/CBFA2, RXR alpha/NR2B1 sFRP-2, SLAIN1 , SOX1, SOX2, SOX9, SOX11, SOX21 , SSEA-1 , SSEA-4, TRAF-4, Vimentin, and ZIC1); (iii) pluripotency stem cell markers (e.g., Oct4, NANOG, Sox2, and Myc); (iv) hematopoietic stem cell markers (e.g., CD34, CD59, and CD90/Thy1); and (v) mesenchymal stem cell markers (e.g., CD105, CD90, CD73, CD44, CD45, CD29, CD166, Stro-1, CD106, and GSTT1).

Since measuring stem cells in vivo is difficult and costly, success of the present composition in ameliorating stem cell exhaustion can also be measured, for example, by making the following preferred in vitro ("lab dish") determinations (as well as in vivo determinations): (i) an increase in the number of stem cells (e.g., by at least 2%, at least 5%, at least 10%, at least 20%, at least 30%, at least 40%, at least 50%, at least 60%, at least 70%, at least 80%, at least 90%, or at least 100%); (ii) increased viability of the stem cells (e.g., by at least 2%, at least 5%, at least 10%, at least 20%, at least 30%, at least 40%, at least 50%, at least 60%, at least 70%, at least 80%, at least 90%, or at least 100%); (iii) an increase in potency of the stem cells in vitro (and in vivo as well) (e.g., by at least 2%, at least 5%, at least 10%, at least 20%, at least 30%, at least 40%, at least 50%, at least 60%, at least 70%, at least 80%, at least 90%, or at least 100%); (iv) an increase in the quality of stem cells, measured for example by an increase in growth of the stem cells, an increase in the ability to proliferate, an increase in the ability to form colonies, or an increase in the ability to produce cells (e.g., by at least 2%, at least 5%, at least 10%, at least 20%, at least 30%, at least 40%, at least 50%, at least 60%, at least 70%, at least 80%, at least 90%, or at least 100%); (v) an increased lifespan of stem cells, as measured in hours (e.g., by at least 2%, at least 5%, at least 10%, at least 20%, at least 30%, at least 40%, at least 50%, at least 60%, at least 70%, at least 80%, at least 90%, or at least 100%); or (vi) an increased resistance of stem cells against cellular stressors (e.g., heat, cold, or toxins) (e.g., by at least 2%, at least 5%, at least 10%, at least 20%, at least 30%, at least 40%, at least 50%, at least 60%, at least 70%, at least 80%, at least 90%, or at least 100%).

The success of the present composition in ameliorating stem cell exhaustion in a subject (e.g., a human subject) can further be measured, for example, by making any of the following determinations in vitro, but also in vivo: (i) improved stem cell function as measured, for example, by replication ability, potency, proliferation capability, survival (as measured, for example, temporally (e.g., number of extra hours of survival), by exposing stem cells to physiological stressors and toxins), potency, and quality (e.g., stem cells have an increased number of replications or can generate/produce larger numbers of cells stemming from the stem cells or display increase cell division markers (e.g., cyclin D1 or increased B-catechin) (e.g., by at least 5%, at least 10%, at least 20%, at least 30%, at least 40%, at least 50%, at least 60%, at least 70%, at least 80%, at least 90%, or at least 100%); (ii) improved stem cell transcriptome (i.e. , the stem cell's transcriptome is more similar to the transcriptome of a younger stem cell or a more functional stem cell) (e.g., by at least 0.5%, at least 1 %, at least 2%, at least 5%, at least 10%, at least 20%, at least 30%, at least 40%, at least 50%, at least 60%, at least 70%, at least 80%, at least 90%, or at least 100%); (iii) improved stem cell proteome (i.e. , the stem cell's proteome is more similar to the proteome of a younger stem cell or more functional stem cell) (e.g., by at least 0.5%, at least 1%, at least 2%, at least 5%, at least 10%, at least 20%, at least 30%, at least 40%, at least 50%, at least 60%, at least 70%, at least 80%, at least 90%, or at least 100%); or (iv) an improved stem cell epigenome (i.e., the stem cell's epigenome is more similar to the epigenome of a younger stem cell or more functional stem cell) (e.g., by at least 0.5%, at least 1%, at least 2%, at least 5%, at least 10%, at least 20%, at least 30%, at least 40%, at least 50%, at least 60%, at least 70%, at least 80%, at least 90%, or at least 100%), as assessed, for example, by measuring methylation or histonylation patterns and comparing them to those of younger, more functional, or more potent stem cells.

The success of the present composition in ameliorating adverse physiological events in a subject (e.g., a human subject) can be measured, for example, by making any of the following determinations: (i) a decrease in the number and/or severity of skin wrinkles as measured, for example, via wrinkle surface area (e.g., via 3D topography or prof i lorn etry); (ii) an increase in skin radiance as measured, for example, via an identometer, cutometer, transepidermal water loss meter, corneometer, or a skin transcriptome or proteome biomarker panel that shows improved skin functioning; (iii) improved cognition as measured, for example, via a cognitive test; (iv) improved feelings of happiness as measured, for example, via the Depressive Symptoms Scale; (v) reduced frailty, as measured, for example, via an international frailty scale or mobility scale (such as the Canadian Study on Health & Aging Clinical Frailty Scale (CSHA-CFS)); (vi) improved reaction time; (vii) improved neuromuscular capability and strength (e.g., increased stamina, improved balance, improved proprioception, improved mobility (measured by 6-minute walk test (6MWT), 4-m gait speed test (4MGST), improved short physical performance battery (SPPB) score, or tests measuring balance, gait speed tests, chair stand tests or other markers of physical function), increased grip strength, increased power, increased activity levels, improved 400 m walk test, and improved arm curl test results); (viii) improved blood flow, as measured by increase of unit of blood per mm3 of tissue; (ix) reduced blood pressure; (x) improved vascular health, as measured, for example, via pulse wave velocity of the blood vessels or flow-mediated vasodilatation; (xi) induced weight loss, a reduction in waist circumference or BMI, or a reduction in abdominal fat volume; (xii) improvement in heart rate variability (HRV); and (xiii) improvement in an Al-assessed facial aging profile (e.g., as measured via facial AI software that for example assesses wrinkle area, blood flow, or skin sagging).

Importantly, if stem cell health is improved and cell senescence is reduced, the various physiological biomarkers discussed in this example will also improve. This is particularly the case with stem cells in that reducing the aging hallmark of stem cell decline (the measurement of which is discussed above) improves physiological biomarkers.

The success of the present composition in ameliorating genomic instability in a subject (e.g., a human subject) can be measured, for example, by determining a decrease in the level of 8-hydroxydeoxyguanosine (8-OHdG), gamma-H2AX, or biomarkers of DNA mutations (e.g., double strand breaks) in the subject (e.g., by at least 5%, at least 10%, at least 20%, at least 30%, at least 40%, at least 50%, at least 60%, at least 70%, at least 80%, at least 90%, or at least 100%).

The success of the present composition in ameliorating deregulated nutrient sensing in a subject (e.g., a human subject) can be measured, for example, by making any of the following determinations: (i) an increase in the level of insulin sensitivity in the subject (e.g., by at least 5%, at least 10%, at least 20%, at least 30%, at least 40%, at least 50%, at least 60%, at least 70%, at least 80%, at least 90%, or at least 100%); or (ii) a decrease in the level of fasting glucose, fasting insulin, LDL, HbA1c, mTOR activity, or mitochondrial reactive oxygen species in the subject (e.g., by at least 5%, at least 10%, at least 20%, at least 30%, at least 40%, at least 50%, at least 60%, at least 70%, at least 80%, at least 90%, or at least 100%).

### EXAMPLES

### EXAMPLE 1:

**Table 1 shows compositions according to an embodiment of the present invention:**

| **Ingredient** | **Unit** | **Per 100 g** | **Per serving** |
|---|---|---|---|
| Nicotinamide (vit B3) | mg | 900 | 54 |
| Vitamin D | ug | 1250 | 75 |
| Vitamin K - optional | ug | 333.33 | 20 |
| Trimethylglycine (betaine) - optional | ug | 8333.33 | 500 |
| Trans-reseveratrol | ug | 2500 | 150 |
| Broccoli sprouts (Broccoplus^{™} ) | mg | 8333.33 | 500 |
| Punica granatum L. extract | mg | 13333.33 | 800 |
| Buckwheat germ-flour (optional) | mg | 33333.33 | 2000 |

Alternative compositions may include those where niacin is replaced by NMN or NR.

### Example 2

Ten human volunteers take a composition as shown in Example 1 for 6 months. In addition, a placebo group, also consisting of 10 volunteers, takes a placebo. The placebo has similar consistency and taste to the effective composition but contains no active ingredients. Each group consists of 5 women and 5 men, over 40. Each volunteer takes the compositionvor the placebo in the morning with water. On day 0, participants are subjected to an analysis to determine biological age. This analysis includes a blood, urine, faeces and sweat analysis, looking for biomarkers linked to (anti-)ageing. A DNA analysis looks at both mutations and SNPs present in participants' DNA, as well as epigenetic markers linked to (anti-)ageing. During the test, participants are subjected to the same test bi-monthly. After six months, the group that took the composition according to Example 1 is found to have measured a significant improvement in biological age as an improvement in biomarkers.

### Example 3

The set-up from Example 2 is repeated, but now the placebo group takes either vitamin D, sulforaphane, or pomegranate extract, each at equal amounts as from the composition from Example 1. The group taking the combination (see example 1) has a biological age significantly lower than the effective age.

### Example 4

The biological age and inflammation status of a female at the age of 40 was measured by means of a commercially available epigenetic test looking at the methylation status of DNA (Muhdo epigenetic kit). Measuring occurred prior to taking a composition as discussed in Example 1. The test subject consistently took the supplement for at least 12 months and this for at least 5 days a week, without any stop. No significant changes to their degree of activity and diet occurred during the test period. After the test period, the test subject was again subjected to the same epigenetic test. Table 1 provides the result. The test subject showed a significant decrease in biological age, age of the eyes and age in memory. Also the inflammation levels considerably dropped. Only the age of the ears showed a negative evolution. After checking the DNA profile of the female patient, it was shown that the female had a genetic disposition for hearing loss, which could have contributed to the decline in age of the ears. Nonetheless, overall the results showed that the composition is effective and has a positive influence on aspects of biological age.

**Table 1**

| **Chronological Age** | **Biological age** | **Age eyes** | **Age ears** | **Age memory** | **Inflammation value** |
|---|---|---|---|---|---|
| 40 | 42.2 | 44.2 | 40.8 | 39.6 | 398 |
| 41 | 41.6 | 37.6 | 58.6 | 41.9 | 317 |

It is assumed that the present invention is not limited to the embodiments described above and that some modifications or changes can be added to the described examples without revaluing the added claims.

## Claims

1. An oral anti-ageing formulation comprising a blend of:
- At least one NAD+ precursor chosen from the group of Niacinamide mononucleotide (NMN), Niacinamide Riboside (NR) and niacin
- At least one stilbenoid
- Pomegranate extract and/or ellagitannins
- Sulforaphane or a source of sulforaphane, and
- Vitamin D.

2. Oral anti-ageing composition according to claim 1 further comprising vitamin K and/or trimethylglycine.

3. Oral anti-ageing composition according to any of the preceding claims, wherein an effective dose of the composition includes
- At least 500 mg per 100 g composition Niacinamide mononucleotide (NMN), niacinamide riboside (NR) and/or niacin
- At least 1250 mg per 100 g of composition stilbenoid, preferably resveratrol and/or pterostilbene
- At least 5000 mg per 100 g composition pomegranate extract and/or elligantans
- At least 1000 ppm per 100 g composition sulforaphane or at least 1000 mg per 100 g composition from a source of sulforaphane
- At least 750 µg per 100 g of composition Vitamin D.

4. Oral anti-ageing composition according to any of the preceding claims, **characterised in, that** the source of sulforaphane is broccoli sprouts, wherein the composition preferably comprises at least 5000 mg per 100 g of composition broccoli sprouts or a concentrate of broccoli sprouts.

5. Oral anti-ageing composition according to any of the preceding claims wherein 1 serving of the composition comprises
- at least 25 µg of a NAD+ precursor chosen from the group of Niacinamide mononucleotide (NMN), Niacinamide Riboside (NR) and niacin
- at least 100 mg of a stilbenoid, preferably chosen from the group of (trans) resveratrol or pterostilbene
- at least 500 mg pomegranate extract
- at least 250 mg of broccoli sprouts, and
- at least 50 µg Vitamin D.

6. Oral anti-ageing composition according to one of the preceding claims, further including pre- and/or probiotics.

7. Oral anti-ageing composition according to any of the preceding claims, further comprising at least one ingredient chosen from the group of fisetin, glycine, Rhodiola rosea, malate, alpha-ketoglutarate, glucosamine sulfate, hyaluronic acid, L-theanine, vitamin C and ginger.

8. Oral anti-ageing composition according to any of the preceding claims, comprising one or more ingredients selected from the group consisting of calcium, sodium, potassium, phosphorus, chromium, vanadium, magnesium, zinc, manganese, selenium, copper, molybdenum, boron, iron (and/or derivatives), lithium or a combination thereof.

9. Oral anti-ageing composition according to any of the preceding claims wherein the composition is formulated as a capsule, tablet, powder, drink, bar, chewable tablet, gel, paste, elixir, syrup, drops or lozenge.

10. Oral anti-ageing composition according to any of the preceding claims wherein the composition is a powder and a daily dose is preferably located between 2 and 20 grams, more preferably between 3 and 10 grams, more preferably between 3 and 7 grams.

11. Oral anti-ageing composition according to claim 9 with the feature, that a dose is packaged in a sachet or stick.

12. Composition according to any of the claims 1 to 11 for use in a method for slowing down the ageing of a subject, preferably a mammal, wherein said composition is taken orally.

13. Composition for use according to claim 12, wherein at least one of the parameters chosen from the group consisting of genomic instability, telomere attrition, an epigenetic alteration, loss of proteostasis, deregulated nutrient sensing, mitochondrial dysfunction, cellular senescence, stem cell exhaustion, and altered intercellular communication is measured, as a marker of the ageing process.

14. Composition according to any of the claims 1 to 11 for use in a method of reducing oxidative stress in mitochondria in the cells of a subject, wherein said composition is taken orally.

15. Composition for use according to claim 12 or 14, wherein the daily dose of the composition comprises
- at least 25 µg of a NAD+ precursor chosen from the group of Niacinamide mononucleotide (NMN), Niacinamide Riboside (NR) and niacin
- at least 100 mg of a stilbenoid, preferably chosen from the group of (trans) resveratrol or pterostilbene
- at least 500 mg pomegranate extract
- at least 250 mg of broccoli sprouts, and
- at least 50 µg Vitamin D.

## Patentansprüche

1. Eine orale Anti-Aging-Formulierung, die eine Mischung aus folgenden Bestandteilen umfasst:
- Mindestens einem NAD+-Vorläufer, ausgewählt aus der Gruppe bestehend aus Niacinamid-Mononukleotid (NMN), Niacinamid-Ribosid (NR) und Niacin
- Mindestens einem Stilbenoid
- Granatapfelextrakt und/oder Ellagitannine
- Sulforaphan oder eine Sulforaphanquelle und
- Vitamin D.

2. Orale Anti-Aging-Zusammensetzung nach Anspruch 1, die ferner Vitamin K und/oder Trimethylglycin umfasst.

3. Orale Anti-Aging-Zusammensetzung nach einem der vorstehenden Ansprüche, wobei eine wirksame Dosis der Zusammensetzung enthalt
- mindestens 500 mg pro 100 g Zusammensetzung Niacinamid-Mononukleotid (NMN), Niacinamid-Ribosid (NR) und/oder Niacin
- mindestens 1250 mg pro 100 g Zusammensetzung Stilbenoid, vorzugsweise Resveratrol und/oder Pterostilben
- mindestens 5000 mg pro 100 g Zusammensetzung Granatapfelextrakt und/oder Elligantane
- Mindestens 1000 ppm pro 100 g Zusammensetzung Sulforaphan oder mindestens 1000 mg pro 100 g Zusammensetzung aus einer Sulforaphanquelle
- Mindestens 750 µg Vitamin D pro 100 g der Zusammensetzung.

4. Orale Anti-Aging-Zusammensetzung nach einem der vorstehenden Ansprüche, **dadurch gekennzeichnet, dass** die Quelle für Sulforaphan Brokkolisprossen sind, wobei die Zusammensetzung vorzugsweise mindestens 5000 mg pro 100 g der Zusammensetzung an Brokkolisprossen oder einem Konzentrat aus Brokkolisprossen umfasst.

5. Orale Anti-Aging-Zusammensetzung nach einem der vorstehenden Ansprüche, wobei 1 Portion der Zusammensetzung umfasst
- mindestens 25 µg eines NAD+-Vorläufers, ausgewählt aus der Gruppe bestehend aus Niacinamid-Mononukleotid (NMN), Niacinamid-Ribosid (NR) und Niacin
- mindestens 100 mg eines Stilbenoids, vorzugsweise ausgewählt aus der Gruppe von (trans)-Resveratrol oder Pterostilben
- mindestens 500 mg Granatapfelextrakt
- mindestens 250 mg Brokkolisprossen und
- mindestens 50 µg Vitamin D.

6. Orale Anti-Aging-Zusammensetzung nach einem der vorstehenden Ansprüche, die ferner Prä- und/oder Probiotika enthält.

7. Orale Anti-Aging-Zusammensetzung nach einem der vorstehenden Ansprüche, die ferner mindestens einen Inhaltsstoff umfasst, der aus der Gruppe ausgewählt ist, die aus Fisetin, Glycin, Rhodiola rosea, Malat, Alpha-Ketoglutarat, Glucosaminsulfat, Hyaluronsäure, L-Theanin, Vitamin C und Ingwer besteht.

8. Orale Anti-Aging-Zusammensetzung nach einem der vorstehenden Ansprüche, die einen oder mehrere Inhaltsstoffe umfasst, ausgewählt aus der Gruppe bestehend aus Calcium, Natrium, Kalium, Phosphor, Chrom, Vanadium, Magnesium, Zink, Mangan, Selen, Kupfer, Molybdän, Bor, Eisen (und/oder Derivaten), Lithium oder einer Kombination davon.

9. Orale Anti-Aging-Zusammensetzung nach einem der vorstehenden Ansprüche, wobei die Zusammensetzung als Kapsel, Tablette, Pulver, Getränk, Riegel, Kautablette, Gel, Paste, Elixier, Sirup, Tropfen oder Lutschtablette formuliert ist.

10. Orale Anti-Aging-Zusammensetzung nach einem der vorstehenden Ansprüche, wobei die Zusammensetzung ein Pulver ist und eine Tagesdosis vorzugsweise zwischen 2 und 20 Gramm, noch bevorzugter zwischen 3 und 10 Gramm, am meisten bevorzugt zwischen 3 und 7 Gramm liegt.

11. Orale Anti-Aging-Zusammensetzung nach Anspruch 9 mit dem Merkmal, dass eine Dosis in einem Beutel oder Stick verpackt ist.

12. Zusammensetzung nach einem der Ansprüche 1 bis 11 zur Verwendung in einem Verfahren zur Verlangsamung der Alterung eines Subjekts, vorzugsweise eines Säugetiers, wobei die Zusammensetzung oral eingenommen wird.

13. Zusammensetzung zur Verwendung nach Anspruch 12, wobei mindestens einer der Parameter, ausgewählt aus der Gruppe bestehend aus genomischer Instabilität, Telomerverkürzung, einer epigenetischen Veränderung, Verlust der Proteostase, deregulierter Nährstoffwahrnehmung, mitochondrialer Dysfunktion, zellulärer Seneszenz, Stammzellerschöpfung und veränderter interzellulärer Kommunikation, als Marker des Alterungsprozesses gemessen wird.

14. Zusammensetzung nach einem der Ansprüche 1 bis 11 zur Verwendung in einem Verfahren zur Verringerung von oxidativem Stress in den Mitochondrien der Zellen eines Subjekts, wobei die Zusammensetzung oral eingenommen wird.

15. Zusammensetzung zur Verwendung nach Anspruch 12 oder 14, wobei die Tagesdosis der Zusammensetzung umfasst
- mindestens 25 µg eines NAD+-Vorläufers, ausgewählt aus der Gruppe bestehend aus Niacinamid-Mononukleotid (NMN), Niacinamid-Ribosid (NR) und Niacin
- mindestens 100 mg eines Stilbenoids, vorzugsweise ausgewählt aus der Gruppe von (trans)-Resveratrol oder Pterostilben
- mindestens 500 mg Granatapfelextrakt
- mindestens 250 mg Brokkolisprossen und
- mindestens 50 µg Vitamin D.

## Revendications

1. Formulation anti-âge orale comprenant un mélange de :
- Au moins un précurseur du NAD+ choisi parmi le groupe comprenant le mononucléotide de niacinamide (NMN), le riboside de niacinamide (NR) et la niacine
- Au moins un stilbénoïde
- d'extrait de grenade et/ou d'ellagitanins
- du sulforaphane ou une source de sulforaphane, et
- de la vitamine D.

2. Composition anti-âge orale selon la revendication 1, comprenant en outre de la vitamine K et/ou de la triméthylglycine.

3. Composition anti-âge orale selon l'une quelconque des revendications précédentes, dans laquelle une dose efficace de la composition contient
- Au moins 500 mg pour 100 g de composition de mononucléotide de niacinamide (NMN), de riboside de niacinamide (NR) et/ou de niacine
- Au moins 1 250 mg par 100 g de composition de stilbénoïde, de préférence de resvératrol et/ou de ptérostilbène
- Au moins 5 000 mg par 100 g de composition d'extrait de grenade et/ou d'elligantanes
- Au moins 1 000 ppm par 100 g de composition de sulforaphane ou au moins 1 000 mg par 100 g de composition provenant d'une source de sulforaphane
- Au moins 750 µg par 100 g de composition de vitamine D.

4. Composition anti-âge orale selon l'une quelconque des revendications précédentes, **caractérisée en ce que** la source de sulforaphane est constituée de pousses de brocoli, la composition comprend de préférence au moins 5000 mg par 100 g de composition de pousses de brocoli ou d'un concentré de pousses de brocoli.

5. Composition anti-âge orale selon l'une quelconque des revendications précédentes, dans laquelle une portion de la composition comprend
- au moins 25 µg d'un précurseur du NAD+ choisi parmi le groupe du mononucléotide de niacinamide (NMN), du riboside de niacinamide (NR) et de la niacine
- au moins 100 mg d'un stilbénoïde, choisi de préférence parmi le groupe du (trans)resvératrol ou du ptérostilbène
- au moins 500 mg d'extrait de grenade
- au moins 250 mg de pousses de brocoli, et
- au moins 50 µg de vitamine D.

6. Composition anti-âge orale selon l'une des revendications précédentes, comprenant en outre des prébiotiques et/ou des probiotiques.

7. Composition anti-âge orale selon l'une quelconque des revendications précédentes, comprenant en outre au moins un ingrédient choisi dans le groupe constitué par la fisétine, la glycine, la Rhodiola rosea, le malate, l'alpha-cétoglutarate, le sulfate de glucosamine, l'acide hyaluronique, la L-théanine, la vitamine C et le gingembre.

8. Composition anti-âge orale selon l'une quelconque des revendications précédentes, comprenant un ou plusieurs ingrédients choisis dans le groupe constitué du calcium, du sodium, du potassium, du phosphore, du chrome, du vanadium, du magnésium, du zinc, du manganèse, du sélénium, du cuivre, du molybdène, du bore, du fer (et/ou de ses dérivés), du lithium ou d'une combinaison de ceux-ci.

9. Composition anti-âge orale selon l'une quelconque des revendications précédentes, dans laquelle la composition est formulée sous forme de capsule, de comprimé, de poudre, de boisson, de barre, de comprimé à croquer, de gel, de pâte, d'élixir, de sirop, de gouttes ou de pastille.

10. Composition anti-âge orale selon l'une quelconque des revendications précédentes, dans laquelle la composition est une poudre et la dose quotidienne est de préférence comprise entre 2 et 20 grammes, de préférence entre 3 et 10 grammes, et plus préférablement entre 3 et 7 grammes.

11. Composition anti-âge orale selon la revendication 9, **caractérisée en ce qu'**une dose est conditionnée dans un sachet ou un stick.

12. Composition selon l'une quelconque des revendications 1 à 11 pour utilisation dans un procédé visant à ralentir le vieillissement d'un sujet, de préférence un mammifère, dans laquelle ladite composition est prise par voie orale.

13. Composition pour utilisation selon la revendication 12, dans laquelle au moins l'un des paramètres choisis parmi le groupe constitué de l'instabilité génomique, l'attrition des télomères, une altération épigénétique, la perte de protéostasie, la dérégulation de la détection des nutriments, le dysfonctionnement mitochondrial, la sénescence cellulaire, l'épuisement des cellules souches et l'altération de la communication intercellulaire altérée est mesuré, en tant que marqueur du processus de vieillissement.

14. Composition selon l'une quelconque des revendications 1 à 11, pour utilisation dans un procédé visant à réduire le stress oxydatif dans les mitochondries des cellules d'un sujet, dans laquelle ladite composition est administrée par voie orale.

15. Composition destinée à être utilisée selon la revendication 12 ou 14, dans laquelle la dose quotidienne de la composition comprend
- au moins 25 µg d'un précurseur du NAD+ choisi parmi le groupe du mononucléotide de niacinamide (NMN), du riboside de niacinamide (NR) et de la niacine
- au moins 100 mg d'un stilbénoïde, choisi de préférence parmi le groupe du (trans)resvératrol ou du ptérostilbène
- au moins 500 mg d'extrait de grenade
- au moins 250 mg de pousses de brocoli, et
- au moins 50 µg de vitamine D.
